# EUROPEAN PATENT APPLICATION

(11) **EP 4 023 202 A1**
(43) Date of publication of application: **06.07.2022**
(21) Application number: 20217491.8
(22) Date of filing: 29.12.2020
(51) Int. Cl.: A61J 1/10, A61K 9/08, B32B 1/00

(54) **MEDICINAL PRODUCT COMPRISING A FLEXIBLE PLASTIC BAG AND AN AQUEOUS, READY-TO-USE SOLUTION OF MIDAZOLAM**

(71) Applicant: B. Braun Melsungen AG, 34212 Melsungen (DE)
(72) Inventor: Robichaud, Jean, J2S 0J9 Saint-Hyacinthe, Quebec (CA); Handfield, Maxim, J2S 0J9 Saint-Hyacinthe, Quebec (CA); Lajoie, Valérie, J2S 0J9 Saint-Hyacinthe, Quebec (CA); Gendron, Marie-Claude, J2S 0J9 Saint-Hyacinthe, Quebec (CA); Fournier, Louis Èric, J2S 0J9 Saint-Hyacinthe, Quebec (CA)
(74) Representative: Patentanwälte Ruff, Wilhelm, Beier, Dauster & Partner mbB

(57) **Abstract**

The invention relates to a medicinal product, in particular sterile medicinal product, comprising a flexible plastic bag, wherein the flexible plastic bag has a wall comprising or consisting of polypropylene and contains an aqueous, ready-to-use solution of midazolam or a pharmaceutically acceptable salt thereof.

## Description

### FIELD OF THE INVENTION

The present invention relates to a medicinal product comprising a flexible plastic bag, wherein the flexible plastic bag contains an aqueous, ready-to-use solution of midazolam.

### BACKGROUND OF THE INVENTION

Midazolam is a benzodiazepine derivative commonly used in intensive care units (ICUs) to control sedation. Chemically, midazolam hydrochloride is 8-chloro-6-(2-fluorophenyl)-1-methyl-4H-imidazo [1,5-a] [1, 4] benzodiazepine hydrochloride. It is a short-acting benzodiazepine central nervous system (CNS) depressant and is generally administered either alone or in combination with second medicament, for example morphine, atropine, scopolamine or meperidine.

It is known that aqueous, ready-to-use solutions of midazolam exhibit a long-term stability for one year when stored in syringes made of polypropylene and at a storage temperature of at most 5 °C (Gilliot et al. "Long-term stability of ready-to-use 1-mg/mL midazolam solution", Am J Health-Syst Pharm., 2020, 77, 681-689).

Further, it is known that midazolam hydrochloride may be stable in an aqueous 5% dextrose solution or aqueous 0.9% sodium chloride solution over 30 days (Hagan et al. "Stability of midazolam hydrochloride in 5% dextrose injection or 0.9% sodium chloride injection over 30 days", Am J Hosp Pharm, 1993; 50; 2379-2381).

Further, it is known that midazolam hydrochloride 1-mg/ml solutions diluted in 5% dextrose injection remain stable over 27 days in both polyolefin and polyvinyl chloride (PVC) bags, regardless of storage condition Karlage et al. "Stability of midazolam hydrochloride injection 1-mg/mL solutions in polyvinyl chloride and polyolefin bags", Am J Health-Syst Pharm; vol 68, Aug 15, 2011; 1537-1540).

WO 2020/170198 A1 discloses a sterile, ready-to-use infusion container comprising a stable, aqueous solution of midazolam or a pharmaceutically acceptable salt thereof. To avoid any adsorption of midazolam and a pharmaceutically acceptable salt thereof, respectively, at least the innermost layer of the wall of the infusion container is made of a cyclo-olefin polymer or a cyclo-olefin copolymer.

The containers storing an aqueous solution of midazolam known from the prior art suffer from the drawbacks that they do not allow a long-term stability at room temperature. As a further challenge remains the stability of midazolam during heat sterilization, in particular using an autoclaving process at 121 °C for 15 minutes.

### OBJECT AND SOLUTION

The object underlying the present invention is therefore to make available a medicinal product containing an aqueous, ready-to-use solution of midazolam or a pharmaceutically acceptable salt thereof, which at least partially avoids the above-mentioned drawbacks.

This object is accomplished by a medicinal product according to independent claim 1. Preferred embodiments of the medicinal product are defined in the dependent claims and the present description. The subject-matter and wording, respectively, of all claims is hereby incorporated into the description by explicit reference.

The medicinal product according to the present invention is preferably a sterile medicinal product.

The medicinal product comprises a flexible, i.e. pliable or soft, plastic bag. Accordingly, the medicinal product according to the present invention is, for example, not in the form of a syringe, vial, ampule, or the like.

The flexible plastic bag has a wall comprising or consisting of polypropylene (PP). Further, the flexible plastic bag comprises or contains an aqueous solution, in particular aqueous, ready-to-use, i.e. pre-mixed, solution, of midazolam or a pharmaceutically acceptable salt thereof, i.e. an aqueous solution, in particular aqueous, ready-to-use, i.e. pre-mixed, solution, comprising or containing midazolam or a pharmaceutically acceptable salt thereof. Preferably, the pharmaceutically acceptable salt of midazolam is midazolam hydrochloride.

The term "plastic bag" as used according to the present invention means a bag comprising or consisting of a plastic.

In particular, the flexible plastic bag according to the present invention may comprise the polypropylene as the only plastic.

Surprisingly, it turned out that a flexible plastic bag, in particular a flexible infusion plastic bag, having a wall comprising or consisting of polypropylene is capable of maintaining stability of an aqueous solution, in particular aqueous, ready-to-use solution, of midazolam or a pharmaceutically acceptable salt thereof for a long term, in particular for 18 months to 24 months, at room temperature. Further, it advantageously turned out that the aqueous solution, in particular aqueous, ready-to-use solution, of midazolam or a pharmaceutically acceptable salt thereof is stable towards heat sterilization, in particular by autoclaving at 121 ° C for 15 minutes, towards unintentional high heat exposure, in particular up to 123 °C and towards applying several freeze-thaw cycles.

The term "ready-to-use solution of midazolam or a pharmaceutically acceptable salt thereof" means a directly available or pre-mixed solution of midazolam or a pharmaceutically acceptable salt thereof, i.e. a solution of midazolam or a pharmaceutically acceptable salt thereof which is ready for use and can be administered without any further preparation or mixing steps, respectively. Preferably, the ready-to-use solution of midazolam or a pharmaceutically acceptable salt thereof is a ready-to-infuse solution of midazolam or a pharmaceutically acceptable salt thereof.

The term "stable" or "stability" as used according to the present invention in terms of the aqueous solution, in particular aqueous, ready-to-use solution, of midazolam or a pharmaceutically acceptable salt thereof preferably means that no turbidity and/or no precipitation, in particular due to formation of degradation products of midazolam or a pharmaceutically acceptable salt thereof, and/or no formation of degradation products of midazolam or of a pharmaceutically acceptable salt thereof occur/occurs under sterilization conditions and/or storage conditions, in particular during storage term and at storage temperature.

The term "polypropylene" as used according to the present invention may mean a polypropylene homopolymer and/or a polypropylene copolymer, in particular a polypropylene random copolymer and/or a polypropylene block copolymer.

The term "polypropylene homopolymer" as used according to the present invention means a polymer which is produced or synthesized by polymerization, in particular chain-growth polymerization, of propene (i.e. no further sort or type of monomer is used for the polymerization).

The term "polypropylene copolymer" as used according to the present invention means a polymer which is produced or synthesized by polymerization, in particular chain-growth polymerization, of propene and at least one further sort or type of monomer such as ethene. In particular, the polypropylene copolymer may be an ethene-propene copolymer (ethylene-propylene copolymer), in particular an ethene-propene block copolymer (ethylene-propylene block copolymer).

The term "room temperature" as used according to the present invention refers to a temperature of 10 °C to 30 °C, in particular 15 °C to 30 °C, preferably 15 °C to 25 °C.

In an embodiment of the invention, the wall of the flexible plastic bag is in the form of a multi-layered wall, i.e. is in the form of a wall being composed or structured of multiple layers. The layers of the multi-layered wall may have the same thickness or may differ in terms of their thickness.

In a further embodiment of the invention, the layers of the multi-layered wall are in the form of co-extruded layers. Advantageously, applying co-extruded layers facilitate formation of the wall of the flexible plastic bag and adhesive or material bonding of the layers in one step.

In a further embodiment of the invention, at least an innermost layer, in particular only an innermost layer, of the multi-layered wall of the flexible plastic bag comprises or consists of the polypropylene.

The term "innermost layer" as used according to the present invention refers to a layer of the multi-layered wall of the flexible plastic bag, which, in particular at least partly, preferably only partly, immediately, i.e. directly, encases or surrounds the aqueous solution, in particular aqueous, ready-to-use solution, of midazolam or a pharmaceutically acceptable salt thereof.

In a further embodiment of the invention, at least a middle layer, in particular only a middle layer, of the multi-layered wall of the flexible plastic bag comprises or consists of the polypropylene.

The term "middle layer" as used according to the present invention refers to a layer, which is arranged, in particular immediately arranged, between an innermost layer and an outermost layer of the multi-layered wall of the flexible plastic bag.

In a further embodiment of the invention, at least an outermost layer, in particular only an outermost layer, of the multi-layered wall of the flexible plastic bag comprises or consists of the polypropylene.

The term "outermost layer" as used according to the present invention refers to a layer of the multi-layered wall of the flexible plastic bag, which separates or defines the flexible plastic bag from its environment or surroundings.

In particular, at least the innermost layer and the middle layer, in particular only the innermost layer and middle layer, of the multi-layered wall of the flexible plastic bag may comprise or consist of the polypropylene.

Alternatively, at least the innermost layer and the outermost layer, in particular only the innermost layer and the outermost layer, of the multi-layered wall of the flexible plastic bag may comprise or consist of the polypropylene.

Alternatively, at least the middle layer and the outermost layer, in particular only the middle layer and the outermost layer, of the multi-layered wall of the flexible plastic bag may comprise or consist of the polypropylene.

In a further embodiment of the invention, all layers of the multi-layered wall of the flexible plastic bag comprise or consist of the polypropylene. In particular, all layers of the multi-layered wall of the flexible plastic bag may comprise the polypropylene as the only plastic.

In particular, the flexible plastic bag may be made of polypropylene based layers, in particular polypropylene based films.

In a further embodiment of the invention, the multi-layered wall of the flexible plastic bag is in the form of a three-layered wall, i.e. being composed of an innermost layer, a middle layer and an outermost layer, in particular as described in the previous and still following description. Preferably, the middle layer is immediately, i.e. directly, arranged or formed on the innermost layer and the outermost layer is immediately, i.e. directly, arranged or formed on the middle layer.

Further, the wall of the flexible plastic bag or the innermost layer and/or middle layer and/or outermost layer of the multi-layered wall of the flexible plastic bag may have a thickness of < 500 µm, in particular of 25 µm to 300 µm, preferably 25 µm to 50 µm or 50 µm to 300 µm. Due to its or their possible thickness, the wall or the layers of the multi-layered wall may also be termed as film or films, according to the present invention.

In a further embodiment of the invention, the polypropylene is selected from the group consisting of isotactic polypropylene, syndiotactic polypropylene, atactic or heterotactic polypropylene and blends of at least two of the afore-said polypropylenes.

Preferably, the polypropylene is a polypropylene homopolymer or a polypropylene copolymer, in particular a polypropylene random copolymer or a polypropylene block copolymer. For example, the polypropylene copolymer may be ethylene-propylene copolymer.

In a further embodiment of the invention, the wall of the flexible plastic bag or the innermost layer and/or middle layer and/or outermost layer of the multi-layered wall of the flexible plastic bag, along the polypropylene, comprises or consists of at least one further polymer. Preferably, the at least one further polymer is selected from the group consisting of polyamide such as polyamide 11, polyvinyl chloride (PVC), ethylene-vinyl acetate (EVA, poly(ethylene-vinyl acetate (PEVA)), polycarbonate (PC), polyethylene (PE), low-density polyethylene (LDPE), high-density polyethylene (HDPE), ethylene propylene copolymer, ethylene alpha olefin copolymer, cyclo-olefin polymer, cyclo-olefin copolymer, styrene-ethylene-butylene (SEB) block copolymer, styrene-ethylene-propylene block copolymer, styrene-ethylene-butylene-styrene block copolymer, styrene-ethylene-butadiene-styrene block copolymer, acrylonitrile butadiene styrene, poly cyclohexane dimethylcyclohexane dicarboxylate elastomer and blends of at least two of the afore-said further polymers. More preferably, the at least one further polymer is styrene-ethylene-butylene (SEB) block copolymer and/or styrene-ethylene-butylene-styrene block copolymer.

For example, at least the innermost layer of the multi-layered wall of the flexible plastic bag may comprise or consist of the at least one further polymer. In particular, only the innermost layer of the multi-layered wall of the flexible plastic bag may comprise or consist of the at least one further polymer, while the middle layer and the outermost layer of the multi-layered wall of the flexible plastic bag may comprise or consist of the polypropylene.

Alternatively, at least the middle layer of the multi-layered wall of the flexible plastic bag may comprise or consist of the at least one further polymer. In particular, only the middle layer of the multi-layered wall of the flexible plastic bag may comprise or consist of the at least one further polymer, while the innermost layer and the outermost layer of the multi-layered wall of the flexible plastic bag may comprise or consist of the polypropylene.

Alternatively, at least the outermost layer of the multi-layered wall of the flexible plastic bag may comprise or consist of the at least one further polymer. In particular, only the outermost layer of the multi-layered wall of the flexible plastic bag may comprise or consist of the at least one further polymer, while the innermost layer and the middle layer of the multi-layered wall of the flexible plastic bag may comprise or consist of the polypropylene.

Alternatively, only the innermost layer and the middle layer of the multi-layered wall of the flexible plastic bag may comprise or consist of the at least one further polymer, while the outermost layer of the multi-layered wall of the flexible plastic bag may comprise or consist of the polypropylene.

Alternatively, only the innermost layer and the outermost layer of the multi-layered wall of the flexible plastic bag may comprise or consist of the at least one further polymer, while the middle layer of the multi-layered wall of the flexible plastic bag may comprise or consist of the polypropylene.

Alternatively, only the middle layer and the outermost layer of the multi-layered wall of the flexible plastic bag may comprise or consist of the at least one further polymer, while the innermost layer of the multi-layered wall of the flexible plastic bag may comprise or consist of the polypropylene.

Further, at least the innermost layer of the multi-layered wall of the flexible plastic bag may comprise or consist of the polypropylene and the at least one further polymer. In particular, only the innermost layer of the multi-layered wall of the flexible plastic bag may comprise or consist of the polypropylene and the at least one further polymer. Preferably, the at least one further polymer is styrene-ethylene-butylene (SEB) block copolymer and/or styrene-ethylene-butylene-styrene block copolymer

Alternatively, at least the middle layer of the multi-layered wall of the flexible plastic bag may comprise or consist of the polypropylene and the at least one further polymer. In particular, only the middle layer of the multi-layered wall of the flexible plastic bag may comprise or consist of the polypropylene and the at least one further polymer. Preferably, the at least one further polymer is styrene-ethylene-butylene (SEB) block copolymer and/or styrene-ethylene-butylene-styrene block copolymer.

Alternatively, at least the outermost layer of the multi-layered wall of the flexible plastic bag may comprise or consist of the polypropylene and the at least one further polymer. In particular, only the outermost layer of the multi-layered wall of the flexible plastic bag may comprise or consist of the polypropylene and the at least one further polymer. Preferably, the at least one further polymer is styrene-ethylene-butylene (SEB) block copolymer and/or styrene-ethylene-butylene-styrene block copolymer.

Alternatively, only the innermost layer and the middle layer of the multi-layered wall of the flexible plastic bag may comprise or consist of the polypropylene and the at least one further polymer. Preferably, the at least one further polymer is styrene-ethylene-butylene (SEB) block copolymer and/or styrene-ethylene-butylene-styrene block copolymer.

Alternatively, only the innermost layer and the outermost layer of the multi-layered wall of the flexible plastic bag may comprise or consist of the polypropylene and the at least one further polymer. Preferably, the at least one further polymer is styrene-ethylene-butylene (SEB) block copolymer and/or styrene-ethylene-butylene-styrene block copolymer.

Alternatively, only the middle layer and the outermost layer of the multi-layered wall of the flexible plastic bag may comprise or consist of the polypropylene and the at least one further polymer. Preferably, the at least one further polymer is styrene-ethylene-butylene (SEB) block copolymer and/or styrene-ethylene-butylene-styrene block copolymer.

Further, at least the innermost layer of the multi-layered wall of the flexible plastic bag may comprise or consist of the polypropylene and the at least one further polymer. In particular, only the innermost layer of the multi-layered wall of the flexible plastic bag may comprise or consist of the polypropylene and the at least one further polymer, while the middle layer and the outermost layer of the multi-layered wall of the flexible plastic bag may comprise or consist of the polypropylene. Preferably, the at least one further polymer is styrene-ethylene-butylene (SEB) block copolymer and/or styrene-ethylene-butylene-styrene block copolymer.

Alternatively, at least the middle layer of the multi-layered wall of the flexible plastic bag may comprise or consist of the polypropylene and the at least one further polymer. In particular, only the middle layer of the multi-layered wall of the flexible plastic bag may comprise or consist of the polypropylene and the at least one further polymer, while the innermost layer and the outermost layer of the multi-layered wall of the flexible plastic bag may comprise or consist of the polypropylene. Preferably, the at least one further polymer is styrene-ethylene-butylene (SEB) block copolymer and/or styrene-ethylene-butylene-styrene block copolymer.

Alternatively, at least the outermost layer of the multi-layered wall of the flexible plastic bag may comprise or consist of the polypropylene and the at least one further polymer. In particular, only the outermost layer of the multi-layered wall of the flexible plastic bag may comprise or consist of the polypropylene and the at least one further polymer, while the innermost layer and the middle layer of the multi-layered wall of the flexible plastic bag may comprise or consist of the polypropylene. Preferably, the at least one further polymer is styrene-ethylene-butylene (SEB) block copolymer and/or styrene-ethylene-butylene-styrene block copolymer.

Alternatively, only the innermost layer and the middle layer of the multi-layered wall of the flexible plastic bag may comprise or consist of the polypropylene and the at least one further polymer, while the outermost layer of the multi-layered wall of the flexible plastic bag may comprise or consist of the polypropylene. Preferably, the at least one further polymer is styrene-ethylene-butylene (SEB) block copolymer and/or styrene-ethylene-butylene-styrene block copolymer.

Alternatively, only the innermost layer and the outermost layer of the multi-layered wall of the flexible plastic bag may comprise or consist of the polypropylene and the at least one further polymer, while the middle layer of the multi-layered wall of the flexible plastic bag may comprise or consist of the polypropylene. Preferably, the at least one further polymer is styrene-ethylene-butylene (SEB) block copolymer and/or styrene-ethylene-butylene-styrene block copolymer.

Alternatively, only the middle layer and the outermost layer of the multi-layered wall of the flexible plastic bag may comprise or consist of the polypropylene and the at least one further polymer, while the innermost layer of the multi-layered wall of the flexible plastic bag may comprise or consist of the polypropylene. Preferably, the at least one further polymer is styrene-ethylene-butylene (SEB) block copolymer and/or styrene-ethylene-butylene-styrene block copolymer.

Alternatively, the innermost layer, middle layer and outermost layer of the multi-layered wall of the flexible plastic bag may comprise or consist of the polypropylene and the at least one further polymer. Preferably, the at least one further polymer is styrene-ethylene-butylene (SEB) block copolymer and/or styrene-ethylene-butylene-styrene block copolymer.

Further, the wall of the flexible plastic bag may be free of cyclo-olefin polymer or cyclo-olefin copolymer.

Further, the wall of the flexible plastic bag is preferably free of metal or metal alloy.

Further, the flexible plastic bag is preferably in the form of a mono-chamber bag, i.e. in the form of a bag having a sole chamber, and the aqueous solution, in particular aqueous, ready-to-use solution, of midazolam or a pharmaceutically acceptable salt thereof is contained in the mono-chamber, i.e. the sole chamber, of the flexible plastic bag.

Further, the flexible plastic bag may be made of a material or may be a bag sold under the commercial name Inerta^{®}. In particular, the flexible plastic bag may be a bag sold under the commercial name Inerta 103^{®} which is a flexible plastic bag having a wall made up of an outermost layer comprising or consisting of polypropylene polymer and styrene-ethylene-butylene (SEB) block copolymer, a middle layer comprising or consisting of polypropylene based polyolefin polymer and styrene-ethylene-butylene (SEB) block copolymer and an innermost layer comprising or consisting of polypropylene based polyolefin polymer and styrene-ethylene-butylene (SEB) block copolymer. Alternatively, the flexible plastic bag may be a bag sold under the commercial name Excel^{®} or Pab^{®} which is a flexible plastic bag having a wall comprising or consisting of ethylene-propylene copolymer.

In a further embodiment of the invention, the flexible plastic bag is overwrapped by an overwrap pouch. Preferably, the overwrap pouch comprises or consists of an opaque material.

The term "opaque material" as used according to the present invention refers to a material which is not transparent for light having a wavelength of 400 nm to 700 nm. For example, the opaque material may be aluminum.

In a further embodiment of the invention, the flexible plastic bag is in the form of a flexible plastic infusion bag.

The term "infusion bag" as used according to the present invention refers to a bag which is typically employed for intravenous administration of medicaments.

Preferably, the flexible plastic bag is adapted to maintain stability of the aqueous solution, in particular aqueous, ready-to-use solution, of midazolam or a pharmaceutically acceptable salt thereof, if the medicinal product is subjected to sterilization, in particular terminal sterilization, preferably by autoclaving, in particular at 121 °C and/or for 15 minutes.

In a further embodiment of the invention, the flexible plastic bag is adapted to maintain stability of the aqueous solution, in particular aqueous, ready-to-use solution, of midazolam or a pharmaceutically acceptable salt thereof during a storage term of 18 months to 24 months, in particular after sterilization, preferably as specified in the preceding paragraph, and at room temperature.

Further, the flexible plastic bag is preferably adapted to maintain stability of the aqueous solution, in particular aqueous, ready-to-use solution, of midazolam or a pharmaceutically acceptable salt thereof, if the medicinal product is exposed to heat, in particular to a temperature of up to 123 °C.

Further, the flexible plastic bag is preferably adapted to maintain stability of the aqueous solution, in particular aqueous, ready-to-use solution, of midazolam or a pharmaceutically acceptable salt thereof, if the medicinal product is subjected to at least one freeze-thaw cycle, in particular to one or several freeze-thaw cycles.

Further, the flexible plastic bag may have an oxygen transmission rate, in particular when measured at 23 °C and 40% relative humidity, of 1000 ml/(m².24 hour.atm) to 1300 ml/(m².24 hour.atm), in particular 1050 ml/(m².24 hour.atm) to 1200 ml/(m².24 hour.atm), preferably 1050 ml/(m².24 hour.atm) to 1150 ml/(m².24 hour.atm). The "oxygen transmission rate", also abbreviated as "OTR", as used according to the present invention may be determined by ASTM D3985 or ISO 15105

Further, the flexible plastic bag may have a water vapor transmission rate of ≤ 3.0 g m⁻² day⁻¹, in particular < 3.0 g m⁻² day⁻¹. Preferably, the flexible plastic bag may have a water vapor transmission rate of 3.0 g m⁻² day⁻¹ to 0 g m⁻² day⁻¹, in particular 2.0 g m⁻² day⁻¹ to 0 g m⁻² day⁻¹, preferably 1.0 g m⁻² day⁻¹ to 0 g m⁻² day⁻¹. The "water vapor transmission rate", also abbreviated as "WVTR", as used according to the present invention may be determined by ASTM F1249 or ISO 15106.

Further, the flexible plastic bag may have a volume capacity of 50 ml to 500 ml, in particular 50 ml to 350 ml, preferably 50 ml to 250 ml.

Further, the flexible plastic bag may not be limited in terms of shape, length and width. However, preferably the flexible plastic bag is rectangular in shape. For example, the flexible plastic bag may have a length of 150 mm to 250 mm and a width of 50 mm to 150 mm.

Further, the aqueous solution, in particular aqueous, ready-to-use solution, of midazolam or a pharmaceutically acceptable salt thereof may have a concentration of midazolam or a pharmaceutically acceptable salt thereof of 0.5 mg/ml to 5 mg/ml, in particular 0.5 mg/ml to 2 mg/ml, preferably 1 mg/ml.

Further, the aqueous solution, in particular aqueous, ready-to-use solution, of midazolam or a pharmaceutically acceptable salt thereof may comprise at least one further ingredient, in particular selected from the group consisting of tonicity adjusting agent, pH adjusting agent, osmotic agents, buffers and combinations of at least two of the afore-said ingredients.

The osmotic agent may be selected from the group consisting of sodium chloride, potassium chloride, mannitol, sorbitol, dextrose, sucrose and mixtures of at least two of the afore-said osmotic agents.

Further, the aqueous solution, in particular aqueous, ready-to-use solution, of midazolam or a pharmaceutically acceptable salt thereof may be free of chelating agents and/or antioxidants and/or stabilizers and/or complexing agents and/or preservatives.

Further, the aqueous solution, in particular aqueous, ready-to-use solution, of midazolam or a pharmaceutically acceptable salt thereof may have a pH value of 2.9 to 4.5, in particular 2.9 to 3.7.

Further features and advantages of the invention will become clear from the following description of preferred embodiments in form of a figure, figure description and examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

### BRIEF DESCRPITION OF THE FIGURE

The figure schematically shows the following:

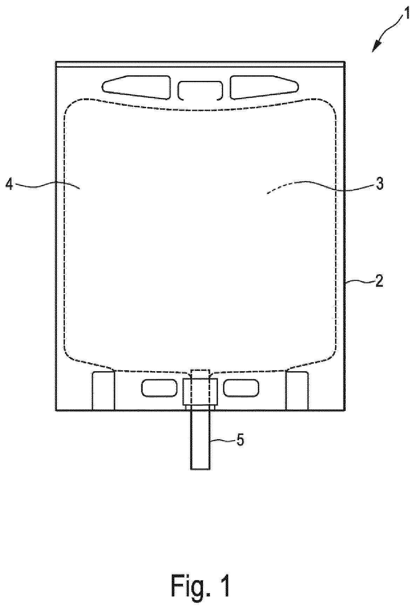

- Fig. 1: an exemplary embodiment of a medicinal product according to the present invention.

### DETAILED FIGURE DESCRPITION

Fig. 1 schematically displays a top view of an embodiment of a medicinal product 1 according to the present invention.

The medicinal product 1 comprises a flexible plastic infusion bag 2 and an aqueous, ready-to-use solution 3 of midazolam or a pharmaceutically acceptable salt thereof. The pharmaceutically acceptable salt of midazolam is preferably midazolam hydrochloride.

The aqueous, ready-to-use solution 3 of midazolam or a pharmaceutically acceptable salt thereof is contained in the flexible plastic infusion bag 2, more specifically in a mono-chamber thereof.

The flexible plastic infusion bag 2 has a wall 4 comprising or consisting of polypropylene. Preferably, the wall 4 is composed of three, in particular three co-extruded, layers, preferably in the form of a film, wherein each layer comprises or consists, in particular is made of, polypropylene. The polypropylene may be isotactic, syndiotactic or atactic/heterotactic polypropylene.

Further, the flexible plastic infusion bag 2 may have at least one port 5. In particular, the flexible plastic bag may have (only) one port, in particular (only) one outlet port, 5 as shown. The at least one port 5 may have a rubber plug to provide a fluid-tight closure of the passage and a lid member that clamps the periphery of the rubber plug. Alternatively, the flexible plastic bag may have two ports, in particular an inlet port and an outlet port.

To the outlet port 5, a sterile infusion set (not shown) may be attached, for example by appropriate means such as a spike that, in particular together with a volumetric pump, allows exit delivery of the aqueous, ready-to-use solution 3 of midazolam or a pharmaceutically acceptable salt thereof from the flexible plastic infusion bag 3 to a patient through the infusion set.

### EXAMPLE SECTION

### 1. Manufacture of an aqueous, ready-to-use solution of midazolam hydrochloride

80% of a batch size of water for injection (WFI) was collected at a temperature of 20 °C to 25 °C in stainless steel 316 L manufacturing tank of suitable capacity. Nitrogen gas was purged to get dissolve oxygen content less than 1 mg/l (ppm), pressure of nitrogen gas and time of purging of flow rate of nitrogen gas were recorded. Sodium chloride was added and dissolved gradually followed by stirring for not less than 10 minutes to ensure complete solubilization and clarity of solution visually. While additional excipients were added as below, the purging was stopped. 1% w/w hydrochloric acid (35 ml/Kg) was added and stirred well before approximately 10 minutes. Midazolam hydrochloride was added slowly into bulk solution with continuous stirring, the dispensing container was rinsed with the dissolved oxygen maintained WFI and added to the bulk solution, rinsing was repeated till all the contents were transferred. The pH was adjusted to 3.0 ± 1 using sufficient quantity of 1% w/w hydrochloric acid and stirred for around 1 hour after pH adjustment to ensure complete dissolution of midazolam, stabilization of pH and until the appearance of the solution was a clear colorless solution. The pH was adjusted at 3.4 ± 0.2 using sufficient quantity of 1% w/w sodium hydroxide solution, and the volume was made up with dissolved oxygen maintained WFI stored at 20 °C to 25 °C. If pH got more than the said range during pH adjustment, then again it was adjusted to 3.4 ± 0.2 using 1% w/w hydrochloric acid. The solution was stirred for 10 minutes until a clear solution was obtained. Nitrogen was purged to get dissolve oxygen content less than 1 mg/l (ppm), and the pH was finally checked to be 3.4 ± 0.2.

The bulk solution was filtered through 0.2 micron polyethersulfone (PES) membrane filter. The filtered bulk solution was filled at standard fill volume in infusion bags made of polypropylene based films. The infusion bags were stoppered with sterile stoppers. The stoppered infusion bags were terminally sterilized by autoclaving at 121 °C for 15 minutes. Finally, the infusion bags were overwrapped using aluminum pouch by overwrapping machine. Optionally, the space between overwrap and bags could be replaced with nitrogen or inert gas.

### 2. Double terminal sterilization cycle

Some of the infusion bags as processed under 1. (midazolam hydrochloride 1 mg/ml in 100 ml infusion bags) were subjected to a double terminal sterilization cycle. Each terminal sterilization cycle was carried out by autoclaving at 121 °C for 15 minutes.

The stability of the aqueous, ready-to-use solution of midazolam hydrochloride was checked immediately after the double terminal sterilization cycle, 12 months after the double terminal sterilization cycle and 18 months after the double terminal sterilization cycle. The results obtained are summarized in the below table 1:

**Tabelle 1: Results concerning the impact of a double terminal sterilization cycle**

| **Test/Parameter** | **Method** | **Specification** | **Double sterilization TP 0** | **Double sterilization TP 12** | **Double sterilization TP 18** |
|---|---|---|---|---|---|
| **Description** | Visual | Clear, colorless liquid in a clear flexible plastic container protected by an opaque overwrap film. | Clear, colorless liquid in a clear flexible plastic container protected by an opaque overwrap film. | Clear, colorless liquid in a clear flexible plastic container protected by an opaque overwrap film. | Clear, colorless liquid in a clear flexible plastic container protected by an opaque overwrap film. |
| **Package Integrity** | Visual | By visually inspecting 3 containers: The containers were not leaking upon manual squeezing and visual inspection. | Meets specifications | Meets specifications | Meets specifications |
| **Assay (mg/mL)** | QCO.1252 In-House USP<621 > | 0.9-1.1 mg/mL | 1.0 mg/mL | 1.0 mg/mL | 1.0 mg/mL |
| **Assay (%)** | | 90.0 - 110.0% of nominal concentration of Midazolam | 99.6% | 98.6% | 98.9% |
| **pH** | USP<791> | 2.5-3.7 | 3.2 | 3.3 | 3.2 |

| **Related substances or degradation substances (HPLC)** | | | | | |
|---|---|---|---|---|---|
| **Ph. Eur. Impurity D** | QCO.1252 In-House USP<621 > | NMT 0.5% | <0.05% | <0.05% | <0.05% |
| **Ph. Eur. Impurity F** | | NMT 0.5% | <0.05% | <0.05% | <0.05% |
| **Ph. Eur. Impurity H** | | NMT 0.5% | <0.05% | <0.05% | <0.05% |
| **Ph. Eur. Impurity B** | | NMT 0.5% | <0.05% | <0.05% | <0.05% |
| **Unknown impurities** | | NMT 0.1 % | 0.05% @ RRT = 0.217 0.05% @ RRT = 1.240 | 0.06% @ RRT = 0.230 | 0.05% @ RRT = 0.234 |
| **Total impurities** | | NMT 1.0% | 0.10% | 0.06% | 0.05% |

The results showed that the flexible plastic infusion bag was not affected by the double terminal sterilization cycle. Further, the stability indicating parameters (assay, pH, flexible plastic bag/closure integrity and degradation products) did not show any significant changes both after 12 months and 18 months. The temperature was at 22 °C ± 2 °C and the room humidity was at 40% ± 20% during storage for the duration of the study. In conclusion, the infusion bags and the aqueous, ready-to-use solution of midazolam hydrochloride contained therein were still stable 18 months following the stress condition.

### 3. High heat terminal sterilization

Some of the infusion bags as processed under 1. were subjected to a high heat terminal sterilization process at 123 °C. Then, the infusion bags were tested in terms of stability immediately after the high heat terminal sterilization process and 12 months and 18 months after the high heat terminal sterilization process. The results obtained are summarized in the below table 2:

**Tabelle 2: Results concerning the impact of high heat terminal sterilization**

| **Test/Parameter** | **Method** | **Specification** | **High heat sample TP 0** | **High heat sample TP 12** | **High heat sample TP 18** |
|---|---|---|---|---|---|
| **Description** | Visual | Clear, colorless liquid in a clear flexible plastic container protected by an opaque | Clear, colorless liquid in a clear flexible plastic container protected by an opaque overwrap | Clear, colorless liquid in a clear flexible plastic container protected by an opaque overwrap film. | Clear, colorless liquid in a clear flexible plastic container protected by an opaque overwrap film. |
| | | overwrap film. | film. | | |
| **Package Integrity** | Visual | By visually inspecting 3 containers: The containers were not leaking upon manual squeezing and visual inspection. | Meets specifications | Meets specifications | Meets specifications |
| **Assay (mg/mL)** | QCO.1252 In-House USP<621 > | 0.9-1.1 mg/mL | 1.0 mg/mL | 1.0 mg/mL | 1.0 mg/mL |
| **Assay (%)** | | 90.0 - 110.0% of nominal concentration of Midazolam Injection USP | 99.8% | 98.6% | 98.9% |
| **pH** | USP<791> | 2.5-3.7 | 3.2 | 3.3 | 3.2 |

| **Related substances or degradation substances (HPLC)** | | | | | |
|---|---|---|---|---|---|
| **Ph. Eur. Impurity D** | QCO.1252 In-House USP<621 > | NMT 0.5% | <0.05% | <0.05% | <0.05% |
| **Ph. Eur. Impurity F** | | NMT 0.5% | <0.05% | <0.05% | <0.05% |
| **Ph. Eur. Impurity H** | | NMT 0.5% | <0.05% | <0.05% | <0.05% |
| **Ph. Eur. Impurity B** | | NMT 0.5% | <0.05% | <0.05% | <0.05% |
| **Unknown impurities** | | NMT 0.1 % | 0.05% @ RRT = 0.218 0.06% @ RRT = 1.239 | 0.06% @ RRT = 0.229 | 0.05% @ RRT = 0.234 |
| **Total impurities** | | NMT 1.0% | 0.11% | 0.06% | 0.05% |

The results confirmed that the infusion bags were not affected by the high heat limit condition during terminal sterilization cycle. The stability indicating parameters (assay, pH, container closure integrity and degradation products) did not show any significant changes neither immediately after the high heat terminal sterilization nor 12 months and 18 months after the high heat terminal sterilization process. The temperature was at 22 °C ± 2 °C and the room humidity (RH) was at 40% ± 20% during storage for the duration of the study. In conclusion, the infusion bags and the aqueous, ready-to-use solution of midazolam hydrochloride contained therein were still stable 18 months following the stress condition.

### 4. Impact of a freeze and thaw cycle

Some of the infusion bags as processed under 1. were subjected to freeze and thaw cycles. The samples were placed in a freezer and let thaw for five complete cycles. Six flexible plastic infusion bags were placed in a freezer for a minimum of 12 hours, then removed from the freezer and left at room temperature for a minimum of 4 hours (until completely thawed). The following day, 12 flexible plastic infusion bags were placed into the freezer (6 initial flexible plastic infusion bags and 6 new flexible plastic infusion bags). These steps were repeated for five consecutive days. Therefore, a total of 30 flexible plastic infusion bags were used for this study.

The object of this study was to determine the impact of the freeze and thaw cycle on the infusion bags. The results obtained are summarized in the below table 3:

**Tabelle 3: Results concerning the impact of freeze and thaw cycles**

| **Test/Parameter** | **Method** | **Specification** | **Not sterilized control sample** | **Sterilized sample** | **Freeze and thaw 5 cycles** |
|---|---|---|---|---|---|
| **Description** | Visual | Clear, colorless liquid in a clear flexible plastic container protected by an opaque overwrap film. | Clear, colorless liquid in a clear flexible plastic container protected by an opaque overwrap film. | Clear, colorless liquid in a clear flexible plastic container protected by an opaque overwrap film. | Clear, colorless liquid in a clear flexible plastic container protected by an opaque overwrap film. |
| **Package Integrity** | Visual | By visually inspecting 3 containers: The containers were not leaking upon manual squeezing and visual inspection. | Meets specifications | Meets specifications | Meets specifications |
| **Assay (mg/mL)** | QCO.1252 In-House USP<621 > | 0.9-1.1 mg/mL | 1.0 mg/mL | 1.0 mg/mL | 1.0 mg/mL |
| **Assay (%)** | | 90.0 - 110.0% of nominal concentration of Midazolam Injection USP | 100.2% | 99.9% | 99.9% |
| **pH** | USP<791> | 2.5-3.7 | 3.2 | 3.3 | 3.3 |

| **Related substances or degradation substances (HPLC)** | | | | | |
|---|---|---|---|---|---|
| **Ph. Eur. Impurity D** | QCO.1252 In-House USP<621 > | NMT 0.5% | <0.05% | <0.05% | <0.05% |
| **Ph. Eur. Impurity F** | | NMT 0.5% | <0.05% | <0.05% | <0.05% |
| **Ph. Eur. Impurity H** | | NMT 0.5% | <0.05% | <0.05% | <0.05% |
| **Ph. Eur. Impurity B** | | NMT 0.5% | <0.05% | <0.05% | <0.05% |
| **Unknown impurities** | | NMT 0.1 % | 0.05% @ RRT = 0.217 0.05% @ RRT = 1.240 | 0.05% @ RRT = 0.217 0.05% @ RRT = 1.240 | 0.05% @ RRT = 0.217 0.09% @ RRT = 1.239 |
| **Total impurities** | | NMT 1.0% | 0.10% | 0.10% | 0.14% |
| **Particulate matter** | USP<788> | N/A | Not Tested | Pass | Pass |
| **Particle count ≥ 10µm** | | NMT 6000/container | Not Tested | 587/container | 653/container |
| **Particle count ≥ 25µm** | | NMT 600/container | Not Tested | 0/container | 27/container |

The results confirmed that five freeze and thaw cycles had no significant impact on the infusion bags and the aqueous, ready-to-use solution of midazolam hydrochloride contained therein.

## Claims

1. Medicinal product, in particular sterile medicinal product, comprising a flexible plastic bag, wherein the flexible plastic bag has a wall comprising or consisting of polypropylene and contains an aqueous, ready-to-use solution of midazolam or a pharmaceutically acceptable salt thereof.

2. Medicinal product according to claim 1, **characterized in that** the wall is in the form of a multi-layered wall.

3. Medicinal product according to claim 2, **characterized in that** the layers of the multi-layered wall are co-extruded layers.

4. Medicinal product bags according to claim 2 or 3, **characterized in that** at least an innermost layer of the multi-layered wall comprises or consists of the polypropylene.

5. Medicinal product according to any of the claim 2 or 4, **characterized in that** at least a middle layer of the multi-layered wall comprises or consists of the polypropylene.

6. Medicinal product according to any of the claims 2 to 5, **characterized in that** at least an outermost layer of the multi-layered wall comprises or consists of the polypropylene.

7. Medicinal product according to any of the claims 2 to 6, **characterized in that** all layers of the multi-layered wall comprise or consist of the polypropylene.

8. Medicinal product according to any of the preceding claims, in particular to any of the claims 2 to 7, **characterized in that** the wall, in particular every layer of the multi-layer wall comprises or consists of the polypropylene.

9. Medicinal product according to any of the claims 2 to 8, **characterized in that** the multi-layered wall is in the form of a three-layered wall.

10. Medicinal product according to any of the preceding claims, **characterized in that** the aqueous, ready-to-use solution of midazolam or a pharmaceutically acceptable salt thereof may have a concentration of midazolam or a pharmaceutically acceptable salt thereof of 0.5 mg/ml to 5 mg/ml, in particular 0.5 mg/ml to 2 mg/ml, preferably 1 mg/ml.

11. Medicinal product according to any of the preceding claims, **characterized in that** the polypropylene is selected from the group consisting of isotactic polypropylene, syndiotactic polypropylene, atactic or heterotactic polypropylene and blends of at least two of the afore-said polypropylenes.

12. Medicinal product according to any of the preceding claims, in particular according to any of the claims 4 to 11, **characterized in that** the wall of the flexible plastic bag, in particular the innermost layer and/or middle layer and/or outermost layer of the multi-layered wall of the flexible plastic bag, along the polypropylene, comprises or consists of at least one further polymer selected from the group consisting of polyamide such as polyamide 11, polyvinyl chloride, ethylene-vinyl acetate, polycarbonate, low density polyethylene, linear low density polyethylene, high density polyethylene, ethylene propylene copolymer, ethylene alpha olefin copolymer, cyclo-olefin polymer, cyclo-olefin copolymer, styrene-ethylene-butylene (SEB) block copolymer, styrene-ethylene-propylene block copolymer, styrene-ethylene-butylene-styrene block copolymer, styrene-ethylene-butadiene-styrene block copolymer, acrylonitrile butadiene styrene, poly cyclohexane dimethylcyclohexane dicarboxylate elastomer and blends of at least two of the afore-said further polymers.

13. Medicinal product according to any of the preceding claims, **characterized in that** the flexible plastic bag is overwrapped by an overwrap pouch, wherein the overwrap pouch preferably comprises or consists of an opaque material such as aluminum.

14. Medicinal product according to any of the preceding claims, **characterized in that** the flexible plastic bag is a flexible plastic infusion bag.

15. Medicinal product according to any of the preceding claims, **characterized in that** the flexible plastic bag is adapted to maintain stability of the aqueous, ready-to-use solution of midazolam or a pharmaceutically acceptable salt thereof during a storage term of 18 months to 24 months and at room temperature.
